# EUROPEAN PATENT APPLICATION

(11) **EP 2 266 546 A1**
(43) Date of publication of application: **29.12.2010**
(21) Application number: 09382089.2
(22) Date of filing: 08.06.2009
(51) Int. Cl.: A61K 9/51, A61K 8/11, A61K 8/73

(54) **Process for the preparation of colloidal systems for the delivery of active compounds**

(71) Applicant: Advancell Advanced in Vitro Cell Technologies,S.A., 08028 Barcelona (ES)
(72) Inventor: Cuesta Regueiro, Ana Belén, E-15782 Santiago de Compostela (ES); Vila Pena, Ana Isabel, E-15782 Santiago de Compostela (ES)
(74) Representative: ABG Patentes, S.L.

(57) **Abstract**

The present invention relates to a process for the preparation of colloidal systems, such as nanocapsules and nanoparticles, which incorporates a homogenization step for reducing particle size.

## Description

### FIELD OF THE INVENTION

The present invention relates to a process for the preparation of colloidal systems, such as nanocapsules and nanoparticles, which incorporates a homogenization step for reducing particle size.

### BACKGROUND

The administration of biologically active molecules for therapeutic purposes presents numerous difficulties, both depending on the route of administration and the physicochemical and morphological characteristics of the molecules. Some drugs, among them are peptides, proteins and polysaccharides are not effectively absorbed through mucosal surfaces due to the limited permeability of the epithelial barriers in the human and animal body.

It has been demonstrated that the incorporation of macromolecules in nanometric-sized systems makes it easier for them to penetrate the epithelial barriers and protects them from being degraded. Thus, the design of nanoparticulate systems capable of interacting with said barriers is presented as a promising strategy in order to achieve the penetration of active ingredients through mucous membranes.

In this sense, important research work has been performed for the purpose of developing colloidal systems which have a positive net charge and which, therefore, allow them to be absorbed via biological environments of cationic character and/or their adhesion to biological membranes (mucous and skin).

Thus, numerous systems based on nanoemulsions and nanocapsules consisting of a lipophilic nucleus and coated with chitosan for the administration of active ingredients by different routes have been described [WO96/37232; Calvo et al., Colloid. Polym. Sci., 1997, 275, 46-53; El-Shabouri, Intern. J. Pharmac., 2002, 249, 101-8; Filipovic-Grcic, J. Microencap., 2001, 18 (1), 3-12]. The incorporation of the hydrophilic chitosan polymer in the aqueous phase confers the positive net charge to said system.

However, some problems of instability have arisen from the use of these nanocapsule systems since their composition causes, during storage for periods longer than one month, the active ingredient to be gradually release from the lipophilic phase.

Document EP1834635A1describes a nanocapsule system comprising a lipophilic nucleous of a phospholipid and an oil and a hydrophilic phase of chitosan, which have, in addition to a great capacity for the association of active lipophilic molecules and their release in the appropriate biological environment, a clearly improved stability both, during its storage and transportation.

All the methods used in the preparation of said nanocapsule systems are based on a solvent-displacing process wherein on mixing a lipophilic phase and a chitosan-containing aqueous phase, nanocapsules are spontaneously formed.

However, one disadvantage derived from this solvent-displacing process is that the organic solvent of the lipophilic phase diffuses to the aqueous phase sweeping along the active ingredient. Thus, in the subsequent solvent evaporation phase and aqueous phase concentration, the active ingredient precipitates forming crystals. Therefore, the organic solvent used highly depends on its facility to diffuse into the aqueous phase. For example, when encapsulating cyclosporine, the use of acetone is required in spite of its toxicity, since the preferred mixture ethanol/methanol rapidly diffuses to the aqueous phase.

In addition, the quantity of oil which can be incorporated into the lipophilic phase is quite limited, since the use of high amounts leads to the formation of independent globules which tend to form aggregates in the surface of the emulsion without being covered by the chitosan. This feature considerably limits the amount of active ingredient to be incorporated into the nanocapsules since said oil constitutes the medium wherein said active ingredient is dissolved.

Another disadvantage of this process is that the suspension formed on mixing lipophilic and aqueous phase is highly diluted, and once nanocapsules are formed, high amounts of organic solvent and water have to be evaporated in subsequent steps. This makes the process non-viable from an industrial point of view since the evaporation process is very time-consuming. Furthermore, high amounts of water are required in order to avoid the destabilization of the formulation, the presence of free oil and the formation of chitosan aggregates after the stir-evaporation process.

Chitosan has also been used as a natural and biocompatible polymer in the formulation of nanoparticules (WO01/32751, WO99/47130). It has also been used in combination with negatively-charge polymers, such as glycosaminoglycanes (WO2004/112758 WO2007/135164) or anionic polysaccharides, such as chondroitin sulphate (W02007/031812).

The method for preparing nanoparticles of chitosan are based on an ionotropic gelification process, wherein on mixing an aqueous solution containing chitosan and another aqueous solution containing the polymer with negative charge and a crosslinking agent, the nanoparticles are spontaneously formed by the electrostatic interaction between the chitosan and the negatively-charge molecule.

However, this process is directly related to the chitosan concentration since the more concentrated is the solution, the larger is the size of the nanoparticles. Therefore, in order to obtain particles in the nanometric range, low concentrations of chitosan are required which negatively affects the yield of the process.

On the other hand, the homogenization technique for preparing nanoparticulate systems has been widely described in the prior art [WO98/07414, WO02/051390, WO03/042251WO01/89522, WO2005/072709, WO2007/069272, WO2007/059118]. Homogenization is a process of reducing the particle size of liquid pharmaceutical products using dynamic high pressure homogenization, to make a dispersion of the active ingredients much more stable for enhanced clinical effectiveness.

However, none of the documents mentioned above describes the homogenization process to prepare colloidal systems, such as nanocapsules comprising a lipophilic nucluos of a phospholipid and an oil surrounded by a hydrophilic phase of chitosan, or nanoparticles of reticulated chitosan, nor suggest the specific advantages that this method confers to said colloidal systems.

In view of these data, it is highly desirable to develop a process for the preparation of said colloidal systems which overcomes the problems derived from the processes described in the state of the art.

### BRIEF DESCRIPTION OF THE INVENTION

The authors of the present invention have developed a new process for the preparation of colloidal systems which incorporates a homogenization step, which overcomes many of the problems derived from the processes of the prior art such as the solvent-displacing process and the classical ionotropic gelification process.

Thus, in contrast to the solvent-displacing process where the nanocapsules are spontaneously formed by mixing lipophilic and aqueous phases, the process of the invention allows encapsulating at least 2% by weight of the active ingredient into the nanocapsules and up to 4% by weight without the formation of precipitated crystals in the nanometric system.

By the process of the invention, the use of the preferred solvent for each active molecule is possible even with much lower quantities, which allows having a high active ingredient concentration and facilitating the preparation of the colloidal systems since the solvent evaporation phase is practically avoided.

Another advantage derived from the process of the invention is that the average effective size of the nanocapsules is significantly reduced when compared to the solvent-displacing process. This feature is really advantageous for the efficacy of the system and therefore for the release of the active ingredient, since the size reduction of the nanocapsules increases the specific surface between the lipophilic phase and the aqueous phase thus improving the contact between the lipophilic phase and the biological environment (skin and mucous) and making easier the release of the active ingredient.

In addition, the process of the invention also allows incorporating higher amounts of oil into the lipophilic phase without the formation of aggregates, which renders the system with a long-term stability and with the possibility of adding higher amounts of lipophilic active ingredient. It is also possible to incorporate higher amounts of chitosan into the aqueous phase, even up to 2% by weight which is of some importance depending on the active ingredient to be incorporated and the indication to be treated. Besides, since water in the initial aqueous solution can be reduce 35% by weight when compared to the solvent-displacing process a much more concentrated formulation can be obtained without destabilizing the system.

In fact, the process of the invention allows increasing the concentration of the emulsion twelve-fold, thus leading to a significantly reduction of the evaporation process which renders the process to be not only more economical but also safer, faster and less cost-effective.

On the other hand, when compared to the classical ionotropic gelification process where nanoparticles are spontaneously formed by mixing two aqueous phases, the process of the invention allows preparing particles in the nanometric range even when the concentration of chitosan is significantly increased.

Therefore, an aspect of the present invention consists of a process for the preparation of colloidal systems of a size less than 1 µm which comprises:
a) providing a lipophilic solution comprising:
   a.1) at least, a phospholipid component; and
   a.2) an oil;
   a.3) a biologically active lipophilic molecule;
b)providing an aqueous solution which comprises:
   b.1) chitosan or a derivative thereof; and
   b.2) a polyoxyalkylenated compound;
c) mixing said lipophilic and aqueous solutions so as to form an emulsion;
d)subjecting said emulsion to a homogenization process to form nanocapsules
   having an average effective particle size of less than 1 µm;
   or alternatively,
e)providing an aqueous solution comprising chitosan or a derivative thereof and, optionally, a polymer selected from glucomannan, hyaluronic acid, a polyoxyalkylenated compound and derivatives thereof; and
f) providing an aqueous solution comprising a crosslinking agent and a biologically
   active molecule;
g)mixing said both aqueous solutions so as to form an agglomerate; and
h)subjecting said agglomerate to a homogenization process to form particles having an average effective particle size of less than 1 µm.

In another embodiment, the present invention relates to colloidal systems obtainable by the process defined above.

### DETAILED DESCRIPTION OF THE INVENTION

In the context of the present invention, the following terms have the meaning detailed below.

The term "colloidal systems" refers to physical entities whose average effective size is less than 1 µm. It includes nanocapsules and nanoparticles. In a particular embodiment of the invention, the average effective size is less than 350 nm, more preferably less than 300 nm, even more preferably less than 250 nm.

The term "nanocapsule" is understood as a nucleus of lipophilic nature surrounded by a hydrophilic phase which comprises hydrophilic polymers with positive charge which "encapsulates" the nucleus. The nanocapsule is dispersed in an aqueous medium, where said aqueous medium may comprise dissolved additional quantities of hydrophilic polymers. The ionic interaction resulting between the different lipophilic and hydrophilic components of the nanocapsule generates characteristic physical entities, which are independent and observable, whose average effective size is less than 1 µm, i.e. an average size between 1 and 999 nm.

By the term "nanoparticle" it is understood a structure formed by the electrostatic interaction between the chitosan and the active ingredient and from the ionotropic gelification of the conjugate obtained by means of the addition of a reticulating agent. The electrostatic interaction resulting between both components of the nanoparticles and the subsequent reticulating generates characteristic physical entities, which are independent and observable, whose average effective size is less than 1 µm, i.e. an average size between 1 and 999 nm.

The term "average effective size" is understood as the average diameter of the colloidal particle which moves in the aqueous medium. The average size of these systems can be measured using standard procedures known by a person skilled in the art, and which are described, for example, in the experimental part below.

By "homogenization process" it is understood any process which allows, by mechanical means, reducing the size of the globules formed in the emulsion resulting from carrying out steps a) to d) of the process of the invention, or reducing the size of the agglomerates formed as a result of effecting steps e) to h). Examples of homogenization process include high pressure homogenization, sonication, high-shear mixing or the application of impact forces or mechanical stress.

### Alternative 1

The process of the invention for the preparation of colloidal systems comprises one alternative comprising the following steps:
a) providing a lipophilic solution comprising:
   a.1) at least, a phospholipid component;
   a.2) an oil; and
   a.3) a biologically active lipophilic molecule;
b) providing an aqueous solution which comprises:
   b.1) chitosan or a derivative thereof; and
   b.2) a polyoxyalkylenated compound;
c) mixing said lipophilic and aqueous solutions so as to form an emulsion;
d) subjecting said emulsion to a homogenization process to form nanocapsules having an average effective particle size of less than 1 µm;

This process leads to the production of colloidal systems in the form of nanocapsules wherein the lipophilic phase constitutes the nucleous of the nanocapsule, this nucleous being surrounded by the hydrophilic phase which provides the surface of the nanocapsule with a positive charge.

The nanocapsules obtained by carrying out steps a) to d) of the process of the invention are **characterized in that** they have an average effective size of less than 1 µm, preferably they have an average size between 1 and 999 nm, preferably between 50 and 800 nm, more preferably between 100 and 300 nm. The average size of the capsules is mainly influenced by the quantity of the phospholipid component (in larger quantities, the resulting size is smaller), by the quantity and molecular weight of the chitosan (the size increases with a greater quantity or molecular weight), the density of the oil used, and by parameters of the homogenization process, such as pressure, temperature or the magnitude of the applied mechanical forces.

Furthermore, the nanocapsules may have a surface charge (measured by zeta potential) attributed to the amine groups of the chitosan, whose magnitude may vary between +1 mV and +70 mV.

### Lipophilic solution

The lipophilic solution is prepared by dissolving at least one phospholipid component, an oil and the biologically active molecule in an organic solvent. The function of the lipophilic solution is that of housing the active lipophilic ingredient, which will later be released in the suitable biological environment.

### Phospholipid component

In the context of the present application, a phospholipid component is understood to be a type of ionic lipid which, in its most simple form, are comprised of a glycerol, whereto two fatty acids (1,2-diacylglycerol) and a phosphate group are bonded. Usually, the phosphate group is bonded by a phosphodiester bond to another group of atoms, which frequently contain nitrogen, such as choline, serine or ethanolamine. Other phospolipids are derived from the aminoalcohol sphingosine or dihydrosphingosine (sphingolipids), and not necessarily from glycerol. The resulting compound has a lipophilic part derived from to the chains of the fatty acids and a hydrophilic part derived from the phosphate group and the electric charge.

The phospholipid component used in the process of the invention is preferably selected from phosphoglycerides, sphingolipids, cephalin and mixtures thereof. The phosphoglycerides, such as phosphatidylcholine, phosphatidylethanolamine, phosphatidylserine, phosphatidylglycerine, phosphatidylinositol, lysophosphatidylcholine and phosphatidic acid, are lipids formed by fatty acids, glycerine, phosphoric acid and alcohol (e.g. choline, serine, ethanolamine, glycerine or inositol). Sphingolipids such as sphingomyelins are formed by sphingosine, a fatty acid, phosphoric acid and an alcohol or aminoalcohol. Cephalin is formed from glycerine, phosphoric acid, two fatty acids and two alcohol bases (choline and sphingosine).

In a preferred embodiment of the invention, the phospholipid component comprises phosphoglycerides. More preferably, it comprises phosphoglycerides selected from phosphatidylcholine, phosphatidylethanolamine, lysophosphatidylcholine and phosphatidic acid or mixtures thereof.

In a preferred variant, the phospholipid component is lecithin. Preferably commercially available soy lecithin is used, although egg lecithin can also be used.

Lecithin is a complex mixture of lipid compounds, most of them being phospholipid compounds, specifically phosphatidylcholine, phosphatidylethanolamine and lysophosphatidylcholine. In general, lecithins are designated by their commercial name and a number which indicated the percentage of phosphatidylcholine in their composition. The percentage of the phosphatidylcholine in lecithins may vary between 35% up to practically 100%, nevertheless the lecithin used is in the present invention has a percentage of phosphatidylcholine between 35% and 75%, preferably between 35% and 55%. The use of lecithins with a very high percentage of phosphatidylcholine (over 80%) is not advisable as it gives rise to the presence of free oil in the nanocapsule suspension. In this regard, there are several lecithins available on the market having a phosphatidylcholine percentage of no more than 80%. Thus, preferably Epikuron 135F, Epikuron 145V, Epikuron 170, Lipoid S30, Lipoid S45 and Lipoid S75 are used.

Likewise, the phosphatidylcholine/phosphatidylethanolamine ratio influences the formation of nanocapsules with suitable stability properties. Thus, it has been demonstrated that the use of Epikuron 135F, wherein the phosphatidylcholine/phosphatidylethanolamine ratio is approximately 7/1 and the proportion of phosphatidylcholine is greater than 35% in the composition, provides the best stability results.

In a particular embodiment of the invention, the proportion of phospholipid is preferably between 1% and 80% by weight with respect to the total weight of the lipophilic solution including the organic solvent, preferably between 5% and 70%, more preferably between 10% and 60% by weight.

### Oil

The oil used in the preparation of the lipophilic solution includes a vegetable-based oil, understanding as such any oil obtainable from a vegetable source. Examples of vegetable-based oils include sunflower oil, corn oil, soy oil, cotton oil, peanut oil, soy oil, evening primrose oil, borage oil, olive oil, coconut oil, palm oil, hempseed oil, apricot kernel or seed oil, wheatgerm oil, corn germ oil, cocoa butter oil, macadamia oil, almond oil, avocado oil, calendula oil, grapeseed oil, sesame oil, jojoba oil or hazelnut oil.

However, in the context of the present invention the term oil also intends to include an oil having one or more unsaturated and/or saturated chain fatty acids or derivatives thereof. Examples of said fatty acids include, although it is not limited to them, saturated fatty acids such as Mygliol, lauric acid, myristic acid, palmitic acid, stearic acid, acacidic acid, carnacilic acid, araquidic acid, behenic acid, lignoceric acid or cerotic acid; monounsaturated fatty acids such as lauroleic acid, palmitoleic acid, oleic acid, vaccenic acid, gadoleic acid, cetoleic acid, or erucic acid; polyunsaturated fatty acids such as linoleic acid, linolenic acid, gamma linolenic acid, stearidonic acid, araquidonic acid, clupanodonic acid or docosahexaenoic acid or mixtures thereof.

Likewise, derivates of these fatty acids can be used, understanding by this definition those compounds with a highly lipophilic character and produced as a consequence of the reaction of the acid group with alcohols or amines, such as, for example, the esters or amides of said fatty acids. In the same way, the definition of fatty acid derivates include those fatty acids, their esters or their amides which have hydroxyl, ether groups or alkyl groups as substituents of the hydrocarbon chain, such as, for example, cerebronic acid, hydroxynervonic acid, ricinoleic acid, tuberculostearic acid or phytanic acid; or those fatty acids which have a cyclic structure in the hydrocarbon chain, such as lactobacillic acid or chaulmogric acid.

In a preferred embodiment of the invention, the oil comprised in the lipophilic solution is castor oil. Said oil is obtained by cold-pressing *Ricinus Communis L.* seeds, although it is commercially available. It has a composition of long-chain fatty acids (C₁₆-C₁₈): 2% (maximum) of palmitic acid, 2.5% (maximum) of stearic acid, 2.5-6.0% of oleic acid, 2.5-7.0% of linoleic acid 1% (maximum) of linolenic acid and 85-92% of ricinoleic acid.

In a particular embodiment, the proportion of the oil is preferably between 5% and 95% by weight with respect to the total weight of the lipophilic solution including the organic solvent, preferably between 20% and 85%, more preferably between 30% and 80% by weight.

### Biologically active lipophilic molecule

The lipophilic solution further comprises at least one biologically active lipophilic molecule. The incorporation of this molecule is performed by dissolution thereof in this lipophilic solution together with the phospholipid component and the oil, irrespective of the order in which the components are added in the solution.

The term "biologically active lipophilic molecule" relates to any substance of lipophilic character which is used in the treatment, cure, prevention or diagnosis of a disease or which is used to improve the physical and mental well-being of humans and animals. These molecules with lipophilic character may include proteins, peptides, lipids, modified oligonucleotides (lipophilized), corticosteroids, lipophilic vitamins, such as A, D, K and E, antifungal agents, bacteriostatic agents, healing agents, antihistaminic agents, anaesthetic agents, antipruritic agents, antipsoriatic agents, antibiotic agents, antiviral agents, antiseptic agents, antiacne agents, depigmenting agents, antiseborrheic agents, immunosuppressant agents, non-steroidal anti-inflammatory drugs (NSAIDs), Capsilum-derived anti-inflammatory drugs.

In a preferred embodiment, the biologically active lipophilic molecule is cyclosporine.

In another preferred embodiment the biologically active lipophilic molecule is a corticosteroid such as, for example, hydrocortisone, prednisone, fluticasone, prednisolone, triamcinolone, triamcinolone acetonide, dexamethasone, betamethasone, beclomethasone, beclomethasone dipropionate.

In a particular embodiment, the proportion of biologically active lipophilic molecule is preferably between 1% and 80% by weight with respect to the total weight of the lipophilic solution including the organic solvent, preferably between 10% and 50%.

The components of the lipophilic solution are dissolved in an organic solvent. In contrast to the solvent-displacing process, the method of the present invention allows using the preferred solvent for each active ingredient without being diffused to the aqueous solution. However, the use of pharmaceutically acceptable organic solvents, such as, for example, ethanol and isopropanol is preferable.

### Hydrophilic solution

The hydrophilic phase is prepared by dissolving the chitosan and polyoxyalkylenated compound in water. The function of this aqueous phase is probably that of coating the nanocapsule, in addition to providing it with a positive charge which allows it to be absorbed through biological environments of cationic character or absorption on them.

### Chitosan

Chitosan is a polymer of natural origin derived from chitin (poly-N-acetyl-D-glucosamine), where an important part of the acetyl groups of the N have been eliminated by hydrolysis. The degree of deacetylation is preferably greater than 40%, more preferably greater than 60%. In a variant it is between 60-98%. It therefore has an aminopolysaccharide structure and cationic character. It comprises the repetition of monomeric units of formula (I): 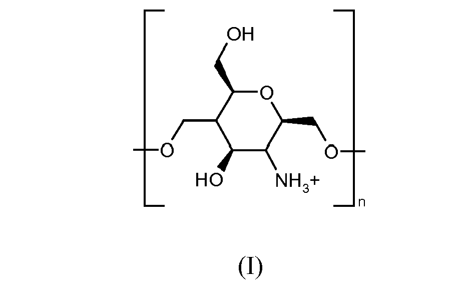 wherein n is an integer, and furthermore m units where the amine group is acetylated. The sum of n+m represents the degree of polymerization, i.e. the number of monomeric units in the chitosan chain.

The chitosan used in the process of the invention for the elaboration of nanocapsules has a molecular weight between 2 and 2000 kDa, preferably between 2 and 500 kDa, more preferably between 5 and 150 kDa. Examples of commercial chitosans which may be used are UP G 113, UP CL 213 and UP CL113 which may be obtained from NovaMatrix, Drammen, Norway; Chitosan Chlorhydrate which may be obtained from Manhtani Chitosan PVT. LTD; KiOmedine-CsU which may be obtained from Kitozyme; Chitopharm S, Chitopharm L and Chitopharma M which may be obtained from Cognis GmbH

As an alternative to chitosan, a derivative thereof can also be used, understanding as such a chitosan wherein one or more hydroxyl groups and/or one or more amine groups have been modified, with the aim of increasing the solubility of the chitosan or increasing the adhesive nature thereof. These derivatives include, among others, acetylated, alkylated or sulfonated chitosans, thiolated derivatives, as is described in Roberts, Chitin Chemistry, Macmillan, 1992, 166. Preferably, when a derivative is used it is selected from O-alkyl ethers, O-acyl esters, trimethyl chitosan, chitosans modified with polyethylene glycol, etc. Other possible derivatives are salts, such as citrate, nitrate, lactate, phosphate, glutamate, etc. In any case, a person skilled in the art knows how to identify the modifications which can be made on the chitosan without affecting the stability and commercial feasibility of the end formulation.

In a particular of the invention, the proportion of chitosan is preferably between 0,1% and 5% by weight with respect to the total weight of the aqueous solution including the water, preferably between 0,2% and 2,5%, more preferably between 0,3% and 1,5% by weight.

### Polyoxyalkylenated compound

By the term "polyoxyalkylenated compound" it is understood a synthetic hydrophilic polymer of non-ionic character having units of alkylene oxide in its structure, being preferred the use of a polyoxyethylene (PEO) or an ethylene oxide-propylene oxide (PEO-PPO) copolymer. When a copolymer of PEO and PPO is used, the proportion of PEO in the copolymer may range from between 10% and 80% by weight, the remaining (up to 100%) being PPO.

These polymers are marketed with different molecular weights, nevertheless, in the present invention the use of polyoxyethylenated derivatives which have molecular weights between 1000 and 25000 is preferred. In a preferred embodiment, the polyoxyethylenated derivative is a triblock copolymer (PEO-PPO-PEO), such as, for example, those commercially called Poloxamer. Good results have been obtained for example with Pluronic F68 (Poloxamer 188).

The polyoxyalkylenated compound is used as surfactant agent in the system and its presence is particularly necessary to confer stability thereto, as otherwise, the nanocapsules aggregate during the storage.

In a particular embodiment, the proportion of the polyoxyalkylenated compound is preferably between 0,1% and 25% by weight with respect to the total weight of the aqueous solution including the water, preferably between 0,5% and 10%, more preferably between 1% and 7,5% by weight.

Once lipophilic and aqueous solutions are prepared, the aqueous solution is heated to a temperature between 30-60° C and the lipophilic solution is heated to a temperature between 40-90°C and then they are mixed. If the lipophilic solution is heated above that maximum, the resulting preparations would not be suitable due to the existence of free oil in the suspension. By contrast, if it is heated under 30°C, the start of solidification of the lipophilic components is observed. This mixing stage can be performed both by adding the aqueous solution to the lipophilic solution, and the lipophilic solution to the aqueous solution, since the addition order does not affect the characteristics of the nanocapsules. On mixing both phases, an oil-in-water emulsion is formed wherein capsules/globules of a size comprised between 20 and 50 µm are dispersed in the aqueous medium.

In a subsequent step, the emulsion thus obtained is subjected to a homogenization process by any method known by a person skilled in the art, in order to reduce the size of the capsules to a nanometric range, i.e. to a size less than 1 µm, and to increase the stability of the system.

Three factors contribute to this enhanced stability of homogenized emulsion: a decrease in the mean diameter of the globules first formed, a decrease in the size distribution of the globules and an increase in density of the globules. Furthermore, a much reduced tendency for clusting is observed.

In a particular embodiment, the homogenization process is carried out by high pressure homogenization, sonication, high-shear mixing or by the application of impact forces or mechanical stress.

In a preferred embodiment of the invention, high pressure homogenization is used. Any equipment known by a skilled person can be used, such as a homogenizator, extruder, microfluidizer or the like.

In a particular embodiment, homogenization process comprises the mechanical treatment of the emulsion formed by passing said emulsion through an orifice in the homogenizator under a differential pressure P1/P2 comprised between 50/800 and 50/3000 bars, depending on size batch equipment.

In a particular embodiment, once the nanocapsules have been formed, the proportion of phospholipid component in the colloidal particle is between 0,5% and 20% by weight with respect to the total weight including water, more preferably between 1% and 10% by weight.

In another particular embodiment, once the nanocapsules have been formed, the proportion of vegetable-based oil in the colloidal particle is between 1% and 20% by weight with respect to the total weight including water, more preferably between 2,5% and 10% by weight.

In another particular embodiment, once the nanocapsules have been formed, the proportion of chitosan in the colloidal particle is between 0,1% and 4% by weight with respect to the total weight including water, more preferably between 0,25% and 2,5% by weight.

In another particular embodiment, once the nanocapsules have been formed, the proportion of polyoxyalkylenated compound in the colloidal particle is between 0,5% and 10% by weight with respect to the total weight including water, more preferably between 1% and 5% by weight.

In another particular embodiment, once the nanocapsules have been formed, the proportion of biologically active lipophilic compound in the colloidal particle is between 0,5% and 20% by weight with respect to the total weight including water, more preferably between 1% and 10% by weight.

Once nanocapsules are formed, additional agents can be added such as viscosing agents, preservatives, fragrance, etc. In a particular embodiment of the invention, viscosing agents are added to the aqueous medium. The addition of these compounds is due to the fact that the nanocapsule dispersed in the aqueous medium results in some cases too fluid from the point of view of its application, especially when manufacturing of semi-solid pharmaceutical or cosmetic compositions such as gels, creams, ointments or balms is required. Among viscosing agents, hydrophilic polymers are particularly preferred. Examples of hydrophilic polymers includes carbohydrates and derivatives, carboxylic acids, poly glycols such as polyvinyl alcohol, derivatives of polyvinylpyrrolidone, chitosan or a derivative thereof, poloxamers, polyethylene glycol, hyaluronic acid celluloses and derivatives or mixtures thereof.

Furthermore, the nanocapsules dispersed in the aqueous phase may be subjected to a high temperature sterilization process without affecting its stability. Said process includes, for example, an autoclaving process, tyndallization, HTST (high temperature short time) and UHT (ultra high temperature). These processes are known by persons skilled in the art.

### Alternative 2

The process of the invention for the preparation of colloidal systems comprises a second alternative comprising the following steps:
e) providing an aqueous solution comprising chitosan or derivatives thereof and, optionally a polymer selected from glucomannan, hyaluronic acid, a polyoxyalkylenated compound and derivatives thereof; and
f) providing an aqueous solution comprising a crosslinking agent and a
   biologically active molecule;
g)mixing said both aqueous solutions so as to form an agglomerate; and
h) subjecting said agglomerate to a homogenization process to form particles having an average effective particle size of less than 1 µm.

This process leads to the production of colloidal systems in the form of nanoparticles, **characterized in that** they have been formed by means of a joint precipitation process of chitosan and the active ingredient in the form of polymeric nanoclusters caused by the addition of a crosslinking agent.

The nanoparticles obtained by carrying out steps e) to h) of the process of the invention are **characterized in that** they have an average effective size of less than 1 µm preferably they have an average size between 1 and 999 nm, preferably between 50 and 800 nm, more preferably between 200 and 700 nm. The average particle size is mainly influenced by the molecular weight of chitosan, by the degree of deacetylation of chitosan, by the proportion of chitosan and also by the particle formation conditions (chitosan concentration, crosslinking agent concentration and ratio between them).

The nanoparticles may have a surface electric charge (measured by zeta potential) which varies depending on the proportion of the chitosan and biologically active molecule. Depending on the chitosan/active molecule proportion and particularly on the degree of deacetylation and on the presence or absence of inorganic salts, the charge magnitude may vary between 0 mV and +60 mV, preferably between +1 and +50 mV.

### Aqueous solutions

The aqueous solution provided in step e) is prepared by dissolving the chitosan in water. The chitosan used in this step are the same as that described previously for alternative 1.

In a particular embodiment, the aqueous solution also comprises a polyoxyethylenated compound which can also be the same as that described previously for alternative 1.

In another particular embodiment, the aqueous solution further comprises hyaluronan understanding as such, a linear polymer which comprises the repetition of a disaccharide structure formed by alternate addition of D-glucuronic acid and D-N-acetylglucosamine, linked together via alternating beta-1,4 and beta-1,3 glycosidic bonds.

The term "hyaluronan" as used in the present description includes either the hyaluronic acid or a conjugate base thereof (hyaluronate). This conjugate base can be an alkali salt of the hyaluronic acid which include inorganic salts such as, for example, sodium, potassium, calcium, ammonium, magnesium, aluminium and lithium salts, and organic salts such as basic aminoacid salts.

The hyaluronan of high molecular weight is commercially available, while that of lower molecular weight can be obtained by fragmentation of hyaluronan of high molecular weight, for example using a hyaluronidase enzyme.

In another particular embodiment, the aqueous solution further comprises glucomannan, understanding as such a mainly straight-chain polymer which comprises the repetition of a saccharide structure formed by addition of D-mannose and D-glucose in a ratio 1.6:1, linked together via beta-1,4 glycosyl linkages. Glucomannan is present in large amounts in the wood of conifers and in smaller amounts in the wood of dicotyledons.

In a particular embodiment, the proportion of chitosan is preferably between 0,1% and 4% by weight with respect to the total weight of the aqueous solution including the water, preferably between 0,2% and 2%, more preferably between 0,5% and 1,2% by weight.

When other polymers selected from a polyoxyethylenated compound, hyaluronan and glucomannan are present, the proportion of chitosan with respect to this other polymer may be very variable, ranging between 1/0,001 and 1/10, preferably between 1/0,1 and 1/4.

On the other hand, the aqueous solution provided in step f) is prepared by dissolving the crosslinking agent and the biologically active molecule in water. The presence of the crosslinking agent allows crosslinking of the chitosan-biologically active molecule conjugate. Furthermore, the crosslinking agent provides the nanoparticles with their size, potential and structural characteristics which make them suitable as an administration system for said active molecule.

In a particular embodiment, the crosslinking agent is an anionic salt allowing reticulation of the chitosan- active molecule conjugate by means of ionic gelling, causing the spontaneous formation of nanoparticles. Preferably a polyphosphate salt is used, sodium tripolyphosphate (TPP) being especially preferred. In a particular embodiment of the aforementioned composition, the crosslinking agent/chitosan weight ratio is comprised between 0,001/1 and 0,5/1, the 0,01/1 and 0,20/1 weight ratio being preferred, which provides formulations with a relatively low polydispersity.

The aqueous solution provided in step f) further comprises a biologically active molecule. The term "biologically active molecule" relates to any substance which is used in the treatment, cure, prevention or diagnosis of a disease or which is used to improve the physical and mental well-being of humans and animals. According to the present invention, the nanoparticles obtained by the process of alternative 2 are suitable for incorporating biologically active molecules irrespective of the solubility characteristics thereof. The association capacity will depend on the molecule incorporated, but in general terms it will be high both for hydrophilic molecules and also for those of marked hydrophobic character. These molecules may include polysaccharides, proteins, peptides, lipids, oligonucleotides, polynucleotides, nucleic acids and mixtures thereof. In a preferred embodiment of the invention the biologically active molecule is a polynucleotide, preferably is a DNA-plasmid, such as pEGFP, pBgal and pSEAP. In another preferred embodiment the biologically active molecule is a polysaccharide such as heparin.

In a particular embodiment, the proportion of the biologically active molecule is preferably between 0,001% and 4% by weight with respect to the total weight of the aqueous solution including the water.

Once both aqueous solutions are provided, they are mixed under stirring. This mixing stage can be performed both by adding the aqueous solution of step e) to the aqueous solution of step f), or the aqueous solution of step f) to the aqueous solution of step e), since the addition order does not affect the characteristics of the nanoparticles. On mixing both phases, an aggregate is formed wherein particles of a size comprised between 50 and 100 µm are dispersed in the aqueous medium

In a subsequent step, the aggregates thus obtained are subjected to a homogenization process by any method known by a person skilled in the art, in order to reduce the size of the particles to a nanometric range, i.e. to a size less than 1 µm.

In a particular embodiment, as in the case of alternative 1, the homogenization process is carried out by high pressure homogenization, sonication, high-shear mixing or by the application of impact forces or mechanical stress.

In a preferred embodiment of the invention, high pressure homogenization is used. Any equipment known by a skilled person can be used, such as a homogenizator, extruder, microfluidizer or the like.

In a particular embodiment, homogenization process comprises the mechanical treatment of the emulsion formed by passing said emulsion through an orifice in the homogenizator under a differential pressure P1/P2 comprised between 50/800 and 50/3000 bars, depending on the size batch equipment.

In another particular embodiment, once the nanoparticles have been formed, the proportion of chitosan in the colloidal particle is between 0,01 % and 4% by weight with respect to the total weight including water, more preferably between 0,1% and 1% by weight.

In another particular embodiment, once the nanoparticles have been formed, the proportion of biologically active compound in the colloidal particle is between 0,01% and 5% by weight with respect to the total weight including water, more preferably between 0,05% and 2% by weight.

Below, some illustrative examples are described which reveal the characteristics and advantages of the invention, however, they should not be interpreted as limiting of the object of the invention as is defined in the claims.

### Examples

As process common to the examples detailed below, the size distribution of nanocapsules and nanoparticles has been performed using photon correlation spectroscopy (PCS; Zeta Sizer, Nano series, Nano-ZS, Malvern Instruments, UK) obtaining average size values of the nanoparticle population.

The chitosan (Protasan UP Cl 113) used in the examples is from NovaMatrix-FMC Biopolymer, the Cyclosporine A from TEVA, the castor oil from Panreac ,the phosphatidylcholine (Epikuron 135F) from Cargill, the poloxamer 188 from BASF Corporation, the acetone from Costoya (J.T. Baker) and the remaining products used come from Sigma Aldrich.

The Homogenizator used for carrying out the homogenization step is a Laboratory Homogenizer niro-soavi Panda 2K.

In addition, other equipment was used: Analytical balance (AND GR 202), pH-meter PB-11 (Sartorious), Laboratory Emulsifier reactor ("BIAGI-5", Lleal, S.A.), Mechanical Overhead Stirrer (RW20DZM, IKA), Laboratory Scale (LBP, Baxtran).

### Example 1. Preparation of chitosan nanocapsules with Cyclosporin A

### 1. Preparation of lipophilic solution

Firstly, 125 g of lecithin (Epikuron 135F), 1 g of butylated hydroxytoluene (BHT), 25 g of benzyl alcohol (BA) and 360 g of castor oil are dissolved in a stainless steel flask under mechanical agitation, using the mechanical Overhead Stirrer, and the heating plate at a temperature of 50°C.

Then, 112.05 g of cyclosporine and 100 g of ethanol are slowly added to the above solution at the time the temperature is increased to 80°C. The agitation continues until all the components are dissolved and part of the ethanol is removed.

### 2. Preparation of aqueous solution

The aqueous solution is prepared by dissolving 25 g of chitosan, 62.5 g of poloxamer and 12.6 g of lactic acid in 4.2 L of water in a reactor vessel at 40°C and under agitation (anchor stirrer).

### 3. Emulsion process.

The lipophilic solution is slowly added on the aqueous solution contained in the reactor vessel, under vacuum and at the highest stirring speed, thus forming an emulsion.

### 4. Homogenization of the emulsion.

The emulsion obtained in the previous step is homogenized by passing it under high pressure through the homogenizer, which results in a decrease in the globule size, thus obtaining the nanocapsules.
1 ratio pressure P1/P2 is 50/1000-1200 bar.

### 5. Viscosization with Hydroxyethyl cellulose (HEC) at 0,5%

Viscosization process is carried out in the emulsifier reactor, by adding the HEC on the homogenized emulsion under high agitation and with vacuum.

The composition of the nanocapsules is shown below:

| *Lipophilic Solution* | % by weight |
|---|---|
| BHT | 0,02 |
| BA | 0,5 |
| Lecithin (Epikuron135F) | 2,50 |
| Castor oil | 7,20 |
| Cyclosporin A | 2,25 |
| Ethanol | <1 (final) |

| *Aqueous Solution* | % by weight |
|---|---|
| Chitosan | 0,5 |
| Poloxamer (Lutrol F68) | 1,25 |
| Lactic acid | <1 (final) |
| Ultrapure water | qsf 100% |

| *Viscosizer* | % by weight |
|---|---|
| HEC | 0,5 |

### Example 2. Evaluation of the percentage of encapsulated active lipophilic molecule in the nanocapsule formulation

Nanocapsules were prepared by solvent-displacing process as following detailed.

### 1. Preparation of lipophilic solution:

Firstly, 250 mg of lecithin (2,5% w/w), 720 mg of castor oil (7,2% w/w) and 225 mg of Cyclosporin A (2,25% w/w) are dissolved in 25ml of acetone in a flask under magnetic agitation and heated in the heating plate at a temperature of 50°C until the disolution of the components.

### 2. Preparation of aqueous solution

The aqueous solution is prepared by dissolving 50 mg of chitosan (0,5% w/w) and 125 mg of poloxamer (1,25%) in 100ml of miliQ water in a flask under magnetic agitation and heated in the heating plate at a temperature of 50°C until the dissolution of the components.

When both solutions are dissolved, the lipophilic solution is added on the aqueous solution under magnetic agitation at the temperature of 50°C.

Once mixing both solutions, the resultant emulsion is concentrated by evaporation to a final volume of 10ml.

In addition, nanocapsules were also prepared according to the process described in example 1.

Once nanocapsules were obtained by both methods, the presence of cyclosporine crystals in the system was subsequently evaluated. Thus, the release of cyclosporine A from the nanocapsules has been visually evaluated, observing if there exists the formation of crystals in the aqueous phase and quantifying the cyclosporine concentration by HPLC (maximum solubility in external phase 16 µg/ml) according to the active ingredient encapsulated. In this way, if the quantity quantified in the external phase is equal to the maximum solubility of active ingredient it is deduced that crystal formation exists, although they are not visible. The results are shown in table I.

| Table I: Comparative characteristics of chitosan nanocapsules with cyclosporine A as active ingredient, prepared by solvent-displacing process and emulsion-homogenization process (Media +/- S.D.) | | | |
|---|---|---|---|
| Composition: Cyclosporine A: 2,25%; Vegetable-based oil: 7,2%; Lecithin: 2,5%; Chitosan: 0,5%; Poloxamer: 1,25% and water 100%. | | | |
| | **Size (nm)** | **Polidispersity Index (PDi)** | **Presence of cyclosporine A crystals** |
| Solvent-displacing process | 581±20 | 0,5 | YES |
| Emulsion-homogenization process | 206±2 | 0,2 | NO |

As can be observed in Table I, the process of the invention based on an emulsion/homogenization process provides nanocapsules with a smaller diameter than those obtained by the solvent-displacing and the absence of cyclosporine A crystal formation, indicating that the molecule remains in the oil nucleus of the nanocapsules without diffusing to the aqueous phase.

### Example 3. Influence of organic solvent in the formation of nanocapsules.

Nanocapsules were obtained according to the methods described in examples 1 and 2 but using different organic solvents and different proportions of cyclosporine. In the case of the solvent-displacing process acetone and a mixture of ethanol and isopropanol were used, whereas in the emulsion/homogenization process, only the preferred solvent for cyclosporine, i.e. ethanol, was used. The results are shown in table II.

| **Tabla II**: Comparative characteristics of chitosan nanocapsules with cyclosporine A as active ingredient, prepared by solvent-displacing process and emulsion-homogenization process (Media +/- S.D.), varying solvent and cyclosporine weight percentage. | | | |
|---|---|---|---|
| | **Cyclosporine (weight %)** | **Organic solvent** | **Results** |
| | 2,0 | Ethanol / Isopropanol | Aggregates |
| | 1,0 | Ethanol / Isopropanol | Aggregates |
| | 0,5 | Ethanol / Isopropanol | Aggregates |
| Solvent-displacing process | 1,0 | Acetone | Nanocapsules 427 ± 8 nm |
| | 0,5 | Acetone | Nanocapsules 464 ± 19 nm |
| | 2,0 | Ethanol | Nanocapsules 289 ± 5 nm |
| | 2,25 | Ethanol | Nanocapsules 206 ± 2 nm |
| Emulsion/Homogenization process | 2,7 | Ethanol | Nanocapsules 272 ± 8 nm |
| | 3,0 | Ethanol | Nanocapsules 218 ± 7 nm |
| | 4,0 | Ethanol | Nanocapsules 318 ± 4 nm |

As can be observed in Table II, by the process of the invention it is possible to use ethanol as organic solvent in the lipophilic phase which allows having a high active ingredient concentration and smallest nanocapsules without the formation of aggregates. An advantage derived from the use of ethanol is that said solvent is a common excipient used in cosmetic and dermatological products for being totally innocuous. In the case of the solvent-displacing process, the use of ethanol is not possible without the formation of aggregates since this solvent diffuses to the aqueous phase.

### Example 4. Evaluation of average effective size of nanocapsules.

Nanocapsules were obtained according to the methods described in examples 1 and 2 but incorporating 0.5% by weight of cyclosporine and 4.8% by weight of castor oil. Subsequently, size distribution of nanocapsules and nanoparticles was measured as indicated above.

| **Table III:** Comparative characteristics of chitosan nanocapsules with cyclosporine A as active ingredient, prepared by solvent-displacing process and emulsion-homogenization process (Media +/- S.D.). | | | |
|---|---|---|---|
| Composition (% weight): Cyclosporine A: 0,5%; Oil: 4,8%; Lecithin: 2,5%; Chitosan: 0,5%; Poloxamer: 1,25 and water 100%. | | | |
| | **Cyclosporine (% by weight)** | **Organic Solvent** | **Size (nm)** |
| Solvent-displacing process | 0,5 | acetone | 485 ± 30 |
| Emulsion/Homogenization process | 0,5 | ethanol | 285 ± 6 |

As can be seen in table III, by the emulsion/homogenization process, the average effective size of the nanocapsules is significantly reduced when compared to the solvent-displacing process. This feature is really advantageous for the efficacy of the system and therefore for the release of the active ingredient, since the size reduction of the nanocapsules increases the specific surface between the lipophilic phase and the aqueous phase, thus improving the contact between the lipophilic phase and the biological environment (skin and mucous) and making easier the release of the active ingredient.

### Example 5. Evaluation of the free oil present in the nanocapsule system

Nanocapsules were obtained according to the methods described in examples 1 and 2 without active ingredient. The presence of cyclosporine crystals as well as the presence of free oil in the system was subsequently evaluated.

| **Table IV:** Comparative characteristics of placebo chitosan nanocapsules, prepared by solvent-displacing process and by emulsion-homogenization process (Media +/- S.D.). | | |
|---|---|---|
| Composition (% weight): Oil: 7,2%; Lecithin: 2,5%; Chitosan: 0,5%; Poloxamer: 1,25 and water 100%. | | |
| | **Size (nm)** | **Free oil after centrifugation** |
| Solvent-displacing process | Aggregates | YES |
| Emulsion/Homogenization process | 206 ± 2 | NO |

As can be seen in table IV, the process of the invention also allows incorporating higher amounts of oil into the lipophilic phase without the formation of aggregates, which renders the system with a long-term stability and with the possibility of adding higher amounts of lipophilic active ingredient.

### Example 6. Influence of chitosan concentration

Nanocapsules were obtained according to the methods described in example 5 but increasing the percentage of chitosan to 1% by weight. The presence of chitosan aggregates when incorporating higher amounts of chitosan to the formulation was evaluated.

| **Table V**: Comparative characteristics of placebo chitosan nanocapsules, prepared by solvent-displacing process and by emulsion-homogenization process (Media +/- S.D.). | | | |
|---|---|---|---|
| Composición: Oil: 7,2%; Lecithin: 2,5%; Chitosan: 1%; Poloxamer: 1,25 and water 100%. | | | |
| | **Size (nm)** | **Chitosan concentration (%, CS)** | **Chitosan aggregates** |
| Solvent-displacing process | Aggregates | 1 | YES |
| Emulsion/Homogenization process | 204 ± 2 | 1 | NO |
| Emulsion/Homogenization process | 300 ± 82 | 2 | NO |

As can be seen in table V, by the process of the invention it is possible to incorporate higher amounts of chitosan, i.e. up to 2% by weight, into the aqueous phase without the formation of aggregates which is of some importance depending on the active ingredient to be incorporated and the indication to be treated.

### Example 7. Influence of water percentage in the nanocapsule system

Nanocapsules were obtained according to the methods described in examples 1 and 2 but reducing the amount of water in the aqueous solution and varying the percentage of cyclosporine incorporated into the nanocapsule system. The results are shown in tables VI and VII.

| **Table VI**: Chitosan-cyclosporine nanocapsules size after decreasing the amount of water and therefore increasing the concentration of formulation components (MEDIA +/- S.D.). | | | |
|---|---|---|---|
| | **Cyclosporine (% weight)** | **Water decrease (% weight)** | **Size (nm)** |
| Emulsion/Homogenization process | 3 | 30 | 272 ± 8 |
| Solvent-displacing process | 3 | 30 | Aggregates |

| **Table VII**: Chitosan-cyclosporine A nanocapsules size (MEDIA +/- S.D.) prepared by emulsion/homogenization process. | | | |
|---|---|---|---|
| | **Cyclosporine (% weight)** | **Water decease (% weight)** | **Size (nm)** |
| Emulsion/Homogenization process | 2,0 | 0-35 | 289,± 4 |
| | 4,0 | 30-35 | 318 ± 4 |
| | 2,9 | 30-35 | 218 ± 7 |

In contrast to the solvent-displacing process, the presence of water in the initial aqueous solution of the emulsion/homogenization process can be reduced 35% by weight, thus obtaining a much more concentrated formulation without destabilizing the system.

### Example 8: Preparation of nanoparticles of chitosan and heparin

### 1. Preparation of the solutions

Twenty-one g of Chitosan were dissolved into a reactor containing 3 L of an acetic acid solution (1%) during about 30 minutes using mechanical stirrer (aprox. 1500 rpm) and room temperature.

Tripolyphosphate sodium (TPP) solution was prepared dissolving 3 g of Tripoliphosphate in water up to a volume of 50 ml with miliQ water at room temperature and magnetic stirrer.

Active polysaccharide solution was prepared by dissolving 50 g of heparin in water up to a volume of 500 ml with miliQ water solution (or Ultrapure water), at room temperature and magnetic stirrer.

The aqueous solution of negative compounds, TPP and heparin, is prepared by mixture in a flask 210 ml of active polysaccharide and 17,5 ml of TPP using magnetic stirrer.

### 2. Preparation of macro aggregates suspension

The reaction process tooks place in the reactor containing the chitosan solution. The TPP-CS solution (227,5 ml) was added to the Chitosan solution (3L) under vacuum (stirring at maximum speed for 15 min) to form an aqueous suspension of macro aggregates (at room temperature).

### 3. Suspension homogenization

In order to reduce the particle size of the suspension to get nanoparticles (1-1000 nm), the formulation is passed through the homogenizer NS1001L-PANDA2K (Niro Soavi S.p.A), which broke the aggregates, and consequently reduces the particle size at a range 0,5-1µm (no up to 1µm) of the aqueous suspension. It is required to pass the formulation through the homogenizer twice, at the pressure of 50/1200-1300 bar.

### 4. A conditioning formulation for spraying-drying

Ascorbic acid is added as adjuvant to the nanoparticles suspension obtained in step 3 under magnetic stirrer and room temperature. It is required 20g of Ascorbic acid for 1 L of formulation.

The formulation is stored at 2-8°C, and it is stable during 3 weeks.

The composition of the formulation to be spry-dried would be:

| **Compound** | **% (w/w)** |
|---|---|
| **Chitosan** | **0,65** |
| **Heparin** | **0,65** |
| **Tripolyphosphate** | **0,03** |
| **Ascorbic acid** | **0,20** |
| **Water (acetic media) q.f.** | **100** |

### Example 9. Influence of chitosan concentration in nanoparticle size.

Nanoparticles were obtained by the ionic gelification process as following detailed.

An anionic solution composed of the active polysaccharide and tripolyphosphate was added to the chitosan cationic solution under mechanical stirring during 5 min (1500 rpm and room temperature). The ratio of the components (CS:Active polysaccharide:TPP) is (1:1:0,015).

The nanoparticles were formed spontaneously (size less than 1 micron). The particle size was Chitosan-concentration dependent, i.e. Chitosan concentration higher than 7,5 mg/ml, the systems tend to aggregate (as described in the table VIII).

In addition, nanoparticles were also prepared according to the process described in example 8 but using different concentrations of chitosan.

Once nanoparticles were obtained by both methods, the particle average size was measured. The results are shown in tables VIII and IX.

**Table VIII: Chitosan nanoparticles prepared by ionic gelification after mixing an aqueous solution of chitosan and an aqueous solution of crosslinking agent.**

| **Chitosan concentration (mg/ml)** | **Size (nm)** | **PdI*** |
|---|---|---|
| 1 | 248 | 0,26 |
| 2,5 | 538 | 0,29 |
| 5 | 665 | 0,54 |
| 7,5 | aggregates | - |
| 8 | aggregates | - |
| 10 | aggregates | - |
| **PdI corresponds to Polydispersity index* | | |

**Table IX: Chitosan nanoparticles prepared by emulsion/homogenization process.**

| **Chitosan concentration (mg/ml)** | **Size (nm)** | **PdI*** |
|---|---|---|
| 10 | 1501 | 0,36 |
| 8 | 897 | 0,33 |
| 7,5 | 803 | 0,35 |
| 5 | 629 | 0,24 |
| **PdI corresponds* | *to Polydispersity* | *index* |

As derivable from the results shown in tables VIII and IX, the process of the invention allows preparing particles in the nanometric range even when the concentration of chitosan is significantly increased.

## Claims

1. A process for the preparation of colloidal systems of a size less than 1 µm which comprises:
a) providing a lipophilic solution comprising:
a.1) at least, a phospholipid component; and
a.2) an oil;
a.3) a biologically active lipophilic molecule;
b) providing an aqueous solution which comprises:
b.1) chitosan or a derivative thereof; and
b.2) a polyoxyalkylenated compound;
c) mixing said lipophilic and aqueous solutions so as to form an emulsion;
d) subjecting said emulsion to a homogenization process to form nanocapsules
having an average effective particle size of less than 1 µm;
or alternatively,
e) providing an aqueous solution comprising chitosan or a derivative thereof and, optionally a polymer selected from glucomannan, hyaluronic acid, a polyoxyalkylenated compound and derivatives thereof,
f) providing an aqueous solution comprising a crosslinking agent and a biologically active molecule;
g) mixing said both aqueous solutions so as to form an agglomerate; and
h) subjecting said agglomerate to a homogenization process to form particles having an average effective particle size of less than 1 µm.

2. The process according to claim 1, wherein the phospholipid component is lecithin.

3. The process according to claims 1 or 2, wherein the oil is castor oil.

4. The process according to any of claims 1 to 3, wherein the polyoxyalkylenated compound is a polyoxyethylene or an ethylene oxide-propylene oxide copolymer.

5. The process according to any of claims 1 to 4, wherein the proportion of phospholipid is between 1% and 80% by weight with respect to the total weight of the lipophilic solution including the organic solvent, preferably between 5% and 70%, more preferably between 10% and 60% by weight.

6. The process according to any of claims 1 to 5, wherein the proportion of oil is between 5% and 95% by weight with respect to the total weight of the lipophilic solution including the organic solvent, preferably between 20% and 85%, more preferably between 30% and 80% by weight.

7. The process according to any of claims 1 to 6, wherein the proportion of the biologically active lipophilic molecule is between 1% and 80% by weight with respect to the total weight of the lipophilic solution including the organic solvent, preferably between 10% and 50%.

8. The process according to any of claims 1 to 7, wherein the biologically active lipophilic molecule is cyclosporine.

9. The process according to any of claims 1 to 8, wherein the proportion of chitosan in step b.1) is between 0,1% and 5% by weight with respect to the total weight of the aqueous solution prepared in step b) including the water, preferably between 0,2% and 2,5%, more preferably between 0,3% and 1,5% by weight.

10. The process according to any of claims 1 to 9, wherein the proportion of the polyoxyalkylenated compound in step b.2) is between 0,1% and 25% by weight with respect to the total weight of the aqueous solution prepared in step b) including the water, preferably between 0,5% and 10%, more preferably between 1% and 7,5% by weight.

11. The process according to any of claims 1 to 10, wherein the colloidal systems resulting from steps a) to d) are nanocapsules.

12. The process according to claim 1, wherein the proportion of chitosan in step e) is between 0,1% and 4% by weight with respect to the total weight of the aqueous solution prepared in step e) including the water, preferably between 0,2% and 2%, more preferably between 0,5% and 1,2% by weight.

13. The process according to claims 1 or 12, wherein the proportion of the biologically active molecule in step f) is between 0,001% and 4% by weight with respect to the total weight of the aqueous solution prepared in step f) including the water.

14. The process according to any of claims 1 and 12 to 13, wherein the colloidal systems resulting from steps e) to h) are nanoparticles.

15. Colloidal systems obtainable by a process as defined in any of claims 1 to 14.
